# EUROPEAN PATENT APPLICATION

(11) **EP 4 151 230 A1**
(43) Date of publication of application: **22.03.2023**
(21) Application number: 21803090.6
(22) Date of filing: 14.05.2021
(51) Int. Cl.: A61K 38/48, A01N 61/00, A01N 63/50, A01P 1/00, A23L 3/3463, A23L 3/3526, A23L 33/17, A61K 9/12, A61K 31/045, A61K 35/742, A61K 47/34, A61K 47/42, A61P 31/12, A61P 31/14, A61P 43/00, C12N 15/31

(54) **ANTIVIRAL AGENT**

(30) Priority: 15.05.2020 JP 2020086137
(71) Applicant: JNC Corporation, Chiyoda-ku Tokyo 100-8105 (JP)
(72) Inventor: YOKOTA, Nana, Yokohama-shi, Kanagawa 236-8605 (JP); NAKANO, Tomomi, Yokohama-shi, Kanagawa 236-8605 (JP); HINATSU, Masako, Yokohama-shi, Kanagawa 236-8605 (JP); HIRAKI, Jun, Yokohama-shi, Kanagawa 236-8605 (JP); FUKUSHI, Hideaki, Yokohama-shi, Kanagawa 236-8605 (JP)
(74) Representative: Becker, Eberhard
(86) International application number: PCT/JP2021/018455
(87) International publication number: WO 2021/230358

(57) **Abstract**

The present invention has revealed that an antiviral agent containing a serine protease such as subtilisin, nattokinase, and trypsin has an effect of inactivating non-envelope type viruses, and that combining such an antiviral agent containing a serine protease with a cationic polymer enhances the inactivation effect of the antiviral agent against non-envelope type viruses. As a result, an antiviral agent that is highly safe, that has a high virus inactivation ability, and that contains a component that is inexpensive in terms the manufacturing cost and a cationic polymer, was discovered.

## Description

### [Technical Field]

The present invention relates to an antiviral agent containing a serine protease and a cationic polymer.

### [Background Art]

Hypochlorous acid and sodium hypochlorite are usually used to inactivate non-enveloped type viruses including *Norovirus,* but their inactivation effects are likely to deteriorate over time and also when organic substances coexist, and a sufficient inactivation effect was not obtained at the time of use in some cases. In addition, sodium hypochlorite cannot be used on the fingers from the viewpoint of safety, and furthermore, methods of use thereof have been restricted due to its corrosiveness with respect to metals.

The inventors of the present invention have so far found an antiviral agent in which a natto extract peptide and a cationic polymer are combined, and have showed that this antiviral agent has a virus inactivation function against non-enveloped type viruses (Patent Literature 1). Thereby, an antiviral agent which exhibits an inactivation effect against non-enveloped type viruses and has high safety that enables application to the fingers and oral administration and ingestion was provided.

The natto extract peptide disclosed in Patent Literature 1 is presumed to be a peptide derived from subtilisin which is a type of serine protease produced by bacteria belonging to the genus *Bacillus,* but subtilisin itself and the natto extract peptide disclosed in Patent Literature 1 are different from each other.

However, the natto extract peptide which is one of the components of the antiviral agent cannot inactivate non-enveloped type viruses by itself alone (Patent Literature 1), and furthermore, although the natto extract peptide is a peptide present in natto, the content thereof is as low as about 1%, and it is required to perform extraction and purification using natto as a starting material in order to obtain the natto extract peptide, leading to the problem of high manufacturing costs.

### [Citation List]

### [Patent Literature]

[Patent Literature 1]
Japanese Patent Laid-Open No. 2019-147778

### [Summary of Invention]

### [Technical Problem]

Therefore, it has been desired to manufacture an antiviral agent that is highly safe and has a high virus inactivation ability while reducing the manufacturing costs thereof.

In addition, it is generally desired in the technical field to conduct continuous studies to further enhance the high virus inactivation ability while maintaining the high level of safety of such an antiviral agent.

In view of the above-mentioned situation, an objective of the present invention is to provide an antiviral agent that is highly safe, has a high virus inactivation ability, and contains a component that is inexpensive in terms of the manufacturing cost and a cationic polymer.

### [Solution to Problem]

As a result of extensive research to achieve the above-mentioned objective, the inventors of the present invention found that an antiviral agent containing a serine protease such as subtilisin, nattokinase, and trypsin has an inactivation function against non-enveloped type viruses, and that combining such an antiviral agent containing a serine protease with a cationic polymer enhances the inactivation function of the antiviral agent against non-enveloped type viruses, and thereby the present invention was completed.

That is, the present application provides the following inventions.
[1] An antiviral agent containing: a serine protease; and a cationic polymer, in which the antiviral agent is used against non-enveloped type viruses.
[2] The antiviral agent according to [1], in which the serine protease is produced from a bacterium belonging to the genus *Bacillus,* a bacterium belonging to the genus *Flavobacterium,* a bacterium belonging to the genus *Arthrobacter,* a bacterium belonging to the genus *Streptomyces,* a bacterium belonging to the genus *Geobacillus,* a bacterium belonging to the genus *Aspergillus,* or a bacterium belonging to the genus *Rhizopus.*
[3] The antiviral agent according to [1] or [2], in which the serine protease is a protein having an amino acid sequence set forth in any one of SEQ ID NOs: 1 to 7, or a protein having an amino acid sequence that is 90% or more identical to the amino acid sequence set forth in any one of SEQ ID NOs: 1 to 7 and having an antiviral activity.
[4] The antiviral agent according to any one of [1] to [3], in which the serine protease is subtilisin or nattokinase.
[5] The antiviral agent according to [1], in which the serine protease is trypsin.
[6] The antiviral agent according to any one of [1] to [5], in which the cationic polymer is polylysine or polyethylenimine.
[7] The antiviral agent according to any one of [1] to [6], in which the non-enveloped type virus is *Norovirus.*
[8] The antiviral agent according to any one of [1] to [7], further containing an alcohol.
[9] The antiviral agent according to any one of [1] to [8], in which the antiviral agent is in a form of an aerosolized agent.
[10] A textile product which is obtained by applying the antiviral agent according to any one of [1] to [8].
[11] A food and drink composition containing the antiviral agent according to any one of [1] to [8].
[12] A food cleaning treatment agent comprising the antiviral agent according to any one of [1] to [8].

### [Advantageous Effects of Invention]

A novel antiviral agent that is highly safe, has a virus inactivation ability, and contains a component that is inexpensive in terms of the manufacturing cost and a cationic polymer is provided, in which the antiviral agent has an enhanced virus inactivation ability and exhibits the stable virus inactivation ability as compared to in the case of using a serine protease alone.

### [Description of Embodiments]

### <Antiviral agent>

An antiviral agent of the present invention is an antiviral agent that contains a serine protease and a cationic polymer, and is used against non-enveloped type viruses.

Examples of the serine protease of the present invention include chymotrypsin-type serine proteases including chymotrypsin and chymotrypsin-like serine proteases, trypsin, elastase, subtilisin, nattokinase, thrombin, and plasmin.

In the present invention, a commercially available product may be used as the serine protease, and the main component may not be a serine protease as long as the serine protease is contained. Examples of commercially available products include those having the following trade names:
Alcalase (registered trademark) 2.4 L FG, Protamex (registered trademark), an alkaline protease produced using a JPBL001 strain, and a protease produced using a JPFV001 strain (all manufactured by Novozymes Japan Ltd.);
Peptidase R, Protease P "Amano" 3SD, Protin SD-AY10, and Proteax (all manufactured by Amano Enzyme Inc.);
Bioprase (registered trademark) OP, Bioprase (registered trademark) 30G, Bioprase (registered trademark) 30L, Bioprase (registered trademark) AL-15FG, Bioprase (registered trademark) APL-30, Bioprase (registered trademark) SP-20FG, Bioprase (registered trademark) XL-416F, Denazyme (registered trademark) PMC SOFTER, Denazyme CPO PEPRICH, and Protease CL-15 (all manufactured by Nagase ChemteX Corporation);
AROASE (registered trademark) XA-10, AROASE (registered trademark) AP-10, and AROASE (registered trademark) NP-10 (all manufactured by Yakult Pharmaceutical Industry Co., Ltd.);
Orientase (registered trademark) 22BF (manufactured by HBI Enzymes Inc.);
Sumizyme (registered trademark) MP (manufactured by SHINNIHON CHEMICALS Corporation);
Maxipro BAP, Maxipro PSP, BakeZyme (registered trademark) B 500 BG, and BakeZyme (registered trademark) PPU 95000 (all manufactured by DSM Nutrition Japan K.K.);
Magnax MT103, ENZYLON SA-100, ENZYLON SA-150P, and ENZYLON NBS-100 (all manufactured by RAKUTO KASEI INDUSTRIAL CO., LTD.);
EFFECTENZ (registered trademark) P 2020, EFFECTENZ (registered trademark) M 2020, EFFECTENZ (registered trademark) P 3020, EFFECTENZ (registered trademark) P 150, Optimase (registered trademark) PR, and Multifect (registered trademark) PR 6L (all manufactured by Danisco Japan Co., Ltd.);
ADMIL (manufactured by GODO SHUSEI CO., LTD.); and
Kokulase (registered trademark) P granules (manufactured by Mitsubishi-Chemical Foods Corporation).

In addition, in the present invention, although not particularly limited, the serine protease is may be a serine protease produced from a bacterium belonging to the genus *Bacillus* such as *Bacillus licheniformis, Bacillus clausii, Bacillus polymyxa, Bacillus subtilis, Bacillus amyloliquefaciens,* and *Bacillus alcalophilus*; a bacterium belonging to the genus *Flavobacterium*; a bacterium belonging to the genus *Arthrobacter*; a bacterium belonging to the genus *Streptomyces*; a bacterium belonging to the genus *Geobacillus* such as *Geobacillus stearothermophilus*; a bacterium belonging to the genus *Aspergillus* such as *Aspergillus oryzae, Aspergillus niger,* and *Aspergillus melleus*; or a bacterium belonging to the genus *Rhizopus*.

In the present invention, the bacterium producing the serine protease is preferably *Bacillus licheniformis*, *Bacillus clausii*, *Bacillus subtilis*, *Bacillus amyloliquefaciens*, *Bacillus alcalophilus*, *Geobacillus stearothermophilus*, and *Aspergillus melleus*. *Bacillus licheniformis* and *Bacillus clausii* are more preferable, and a protease produced from these bacteria exhibits a strong and stable antiviral activity. In addition, examples of *Bacillus subtilis* include *Bacillus subtilis* var. natto.

Although not particularly limited, in the present invention, the serine protease may be a protein having an amino acid sequence set forth in any one of SEQ ID NOs: 1 to 7, or may be a protein having an antiviral activity and having an amino acid sequence that is 90% or more identical to the amino acid sequence set forth in any one of SEQ ID NOs: 1 to 7, preferably an amino acid sequence that is 95% or more identical thereto, and more preferably an amino acid sequence that is 98% or more identical thereto.

The protein having the amino acid sequence set forth in SEQ ID NO: 1 is a serine protease produced from *Bacillus subtilis* var. natto, the protein having the amino acid sequence set forth in SEQ ID NO: 2 is a serine protease produced from *Bacillus subtilis* var. natto, the protein having the amino acid sequence set forth in SEQ ID NO: 3 is a serine protease produced from *Bacillus subtilis,* the protein having the amino acid sequence set forth in SEQ ID NO: 4 is a serine protease produced from *Bacillus licheniformis*, the protein having the amino acid sequence set forth in SEQ ID NO: 5 is a serine protease produced from *Bacillus clausii,* the protein having the amino acid sequence set forth in SEQ ID NO: 6 is a serine protease produced from *Bacillus amyloliquefaciens*, and the protein having the amino acid sequence set forth in SEQ ID NO: 7 is a serine protease produced from *Geobacillus stearothermophilus*.

As a method for calculating the numerical value of the identity of the amino acid sequence, a method known to those skilled in the art can be used. For example, calculation by the defined parameters of blastp used in amino acid homology search provided by BLAST (registered trademark) is possible.

In addition, although not particularly limited, in the present invention, the serine protease is preferably a chymotrypsin-type serine protease including chymotrypsin and chymotrypsin-like serine proteases, trypsin, elastase, subtilisin, nattokinase, thrombin, or plasmin. A subtilisin-type serine protease is more preferable, and specifically, there is subtilisin and nattokinase.

The identity of the amino acid sequences between the subtilisin having the amino acid sequence set forth in SEQ ID NO: 1 produced from *Bacillus subtilis* var. natto and nattokinase having the amino acid sequence set forth in SEQ ID NO: 2 produced from *Bacillus subtilis* var. natto is about 99%. Because serine proteases such as subtilisin and nattokinase can be mass-produced by industrial methods, they are superior from the viewpoint that the manufacturing cost is lower than that of natto extract peptides which are not mass-produced by industrial methods.

Trypsin is a safe enzyme known to be usable in foods, and can be obtained inexpensively because it can be mass-produced by industrial methods. Furthermore, the amino acid sequence of trypsin is not particularly limited, and may be any amino acid sequence.

In the present invention, the cationic polymer is not particularly limited as long as it enhances the virus inactivation function of the antiviral agent, but is preferably polylysine or polyethylenimine.

Although not particularly limited, the case in which polylysine is used as the cationic polymer of the present invention is highly safe because polylysine is a naturally occurring polymer.

Polylysine is not particularly limited and may be any of α-polylysine or ε-polylysine, but ε-polylysine is preferable because of its low toxicity and easy availability. A polymer of L-lysine is also generally used.

ε-Polylysine can be manufactured by the method disclosed in Japanese Patent No. 1245361, for example.

The weight-average molecular weight of the polylysine is not particularly limited, but it is preferably 3,000 or more, and more preferably 4,000 or more, is preferably 10,000 or less, more preferably 8,000 or less, and further preferably 6,000 or less, and is particularly preferably in the range of 3,000 to 6,000.

The weight-average molecular weight of the polyethylenimine is not particularly limited, but it is preferably 300 to 10,000,000.

The weight-average molecular weight herein is a value measured by a GPC-LALLS method.

In addition, the cationic polymer of the present invention is usually a homopolymer, but may contain other amino acids as monomers as long as the effect of the present invention is not impaired.

In addition, the cationic polymer of the present invention may be in a free form, or may be in a form of salts of at least one inorganic acid selected from hydrochloric acid, sulfuric acid, phosphoric acid, and hydrobromic acid, or at least one organic acid selected from acetic acid, propionic acid, fumaric acid, malic acid, and citric acid.

As long as the antiviral agent exhibits an antiviral effect, the content ratio between the serine protease and the cationic polymer in the antiviral agent of the present invention is not particularly limited, but is preferably 1:100 to 100:1 as the mass ratio at the time of use.

As long as the antiviral agent exhibits an antiviral effect, the final pH of a formulation containing the antiviral agent of the present invention is not particularly limited, but it is preferably pH 6.0 or more, more preferably pH 7.0 to 11.0, and further preferably pH 7.3 to 10.3, and may be appropriately adjusted according to the type and concentration of the target virus, or may be adjusted according to the application.

The antiviral agent of the present invention is intended to inactivate non-enveloped type viruses. As a result of inactivating viruses, the infectivity of the viruses can be attenuated, or viral infection can be inhibited. Because the antiviral activity of the antiviral agent according to the present invention is not reduced even when organic substances coexist unlike hypochlorous acid or sodium hypochlorite, the antiviral agent is also excellent from the viewpoint that the range of selections for applicable targets, conditions, environments, forms, and the like is much wider than that of hypochlorous acid.

The non-enveloped type viruses on which the antiviral agent of the present invention acts are not particularly limited, but examples thereof include *Norovirus*, *Rotavirus*, *Poliovirus*, *Adenovirus*, and *Feline calicivirus,* where *Norovirus* is preferable.

In the present specification, the "antiviral activity" is evaluated by a plaque assay method or a TCID₅₀ (50% median tissue culture infectious dose) method, which are methods known to those skilled in the art, for example.

In the case of the plaque assay, it is determined that a sample has an antiviral activity when plaque forming units (PFU) when the sample is added are smaller, more preferably 10 times smaller, and further preferably 100 times smaller than when the sample is not added.

In the case of the TCID₅₀ method, it is determined that a sample has an antiviral activity when a log TCID₅₀ value when the sample is added is smaller, more preferably smaller by 1 or more, and further preferably smaller by 2 or more than when the sample is not added, that is, when an antiviral activity value is more than 0, preferably 1 or more, and more preferably 2 or more than when the sample is not added.

Because it is difficult to culture human *Norovirus* in an inactivation test against non-enveloped type viruses, evaluation can be made by testing against *Feline calicivirus* which is generally used as an alternative test. Furthermore, *Murine norovirus* instead of the human *Norovirus* can be used as an alternative test.

Another aspect of the present invention is use of the serine protease and the cationic polymer for inactivation of non-enveloped type viruses.

Still another aspect is use of the serine protease and the cationic polymer in the manufacture of the antiviral agent for inactivation of non-enveloped type viruses.

Still another aspect is the serine protease and the cationic polymer which are used for inactivation of non-enveloped type viruses.

Still another aspect is a method for inactivating non-enveloped type viruses in a subject, the method including application of the serine protease and the cationic polymer to the subject. Examples of the application of the serine protease to the subject include causing a mammal such as a human to orally ingest the serine protease and the cationic polymer as a food and drink composition, bringing into contact or mixing the serine protease and the cationic polymer with foods and drinks, and applying the serine protease and the cationic polymer to textile products.

### <Food and drink composition>

Since the antiviral agent of the present invention can be orally ingested, it can have the form capable of being contained in a food and drink composition.

Examples of aspects of the food and drink composition of the present invention include, but are not particularly limited to, ordinary foods, drinks, food additives, foods with function claims, foods with health claims such as foods for specified health uses, and supplements. The supplements may be solid supplements such as powders, granules, tablets, capsules, and the like, or may be liquid supplements such as solutions, syrups, suspensions, emulsions, and the like, for example.

The form of the food and drink composition may be any of liquid, paste, solid, powder, and the like, and may be tablets, liquid foods, feeds, or the like.

In the case of such a form, ingredients, which are usually added at the time of manufacturing foods and drinks, such as proteins, carbohydrates, fats, nutrients, seasonings, and flavorings can be used in addition to the antiviral agent of the present invention. Examples of the carbohydrates include monosaccharides such as glucose and fructose; disaccharides such as maltose, sucrose, and oligosaccharides; polysaccharides such as normal sugars such as dextrin and cyclodextrin; and sugar alcohols such as xylitol, sorbitol, and erythritol. As the flavoring, natural flavorings (such as thaumatin and stevia extract) and synthetic flavorings (such as saccharin and aspartame) can be used.

In addition, the food and drink composition may further contain, as an additive, excipients, binders, disintegrants, lubricants, stabilizers, corrigents, odor eliminators, pH adjusters, and colorants, which are also added to ordinary foods and drinks.

Examples of the excipients include sugar derivatives such as lactose, sucrose, glucose, mannitol, and sorbitol; starch derivatives such as corn starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethylcellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate.

Examples of the binders include gelatin, polyvinylpyrrolidone, and macrogol, in addition to the above-mentioned excipients.

Examples of the disintegrants include cellulose derivatives or chemically modified starch such as croscarmellose sodium, sodium carboxymethyl starch, and crosslinked polyvinylpyrrolidone, in addition to the above-mentioned excipients.

Examples of the lubricants include talc; stearic acid; stearic acid metal salts such as calcium stearate and magnesium stearate; colloidal silica; waxes such as pea gum and spermaceti; boric acid; glycol; carboxylic acids such as fumaric acid and adipic acid; carboxylic acid sodium salts such as sodium benzoate; sulfates such as sodium sulfate; leucine; lauryl sulfates such as sodium lauryl sulfate and magnesium lauryl sulfate; silicic acids such as anhydrous silicic acid and silicic acid hydrate; and starch derivatives.

Examples of the stabilizers include parahydroxybenzoate esters such as methylparaben and propylparaben; alcohols such as chlorobutanol, benzyl alcohol, and phenylethyl alcohol; benzalkonium chloride; acetic anhydride; sorbic acid; and divalent inorganic salts such as CaCl₂.

Examples of the corrigents and the odor eliminators include sweeteners, acidulants, and fragrances.

A carrier used in the case of a liquid formulation is not particularly limited, but examples thereof include a solvent such as water.

In addition, one form of the food and drink composition may be a form containing other general foods and drinks, and the antiviral agent of the present invention may be added to, for example, wheat flour products such as bread, macaroni, spaghetti, noodles, cake mixes, fried chicken flour, and bread crumbs; instant foods such as instant noodles, cup noodles, retort and cooked foods, cooked canned foods, microwave foods, instant soup and stew, instant miso soup and Japanese soup, canned soup, and freeze-dried foods; processed agricultural products such as canned agricultural products, canned fruits, jams and marmalades, pickles, boiled beans, dried agricultural products, and cereals (processed grain products); processed marine products such as canned marine products, fish meat hams and sausages, marine product paste cakes, marine product jerky, and marine products boiled and flavored with soy sauce and sugar; processed livestock products such as canned livestock products and livestock pastes, and livestock meat hams and sausages; milk and dairy products such as processed milk, milk drinks, yogurts, lactic acid drinks, cheese, ice creams, formulated milk powders, cream, and other dairy products; fats and oils such as butter, margarines, and vegetable oils; basic seasonings such as soy sauce, miso, sauces, processed tomato seasonings, mirin, and vinegar; complex seasonings and foods such as cooking mixes, curry ingredients, mixed sauces, dressings, Japanese soup bases used in noodle dishes, spices, and other complex seasonings; frozen foods such as material frozen foods, half-cooked frozen foods, and cooked frozen foods; confectionery such as caramels, candies, chewing gums, chocolates, cookies, biscuits, cakes, pies, snacks, crackers, Japanese confectionery, rice confectionery, bean confectionery, and dessert confectionery; drinks such as carbonated drinks, natural fruit juices, fruit juice drinks, soft drinks containing fruit juices, fruit pulp drinks, fruit drinks containing shredded fruit, vegetable drinks, soy milk, soy milk drinks, coffee drinks, tea drinks, powdered drinks, concentrated drinks, sports drinks, nutritional drinks, and alcoholic drinks; and foods and drinks other than the above examples.

Furthermore, as one form of the food and drink composition, there is a food cleaning treatment agent which is used for performing antiviral treatment on foods and drinks including fresh foods to prevent the occurrence of food poisoning. For example, although there is no particular limitation, an aspect in which raw oysters and the like contaminated with *Norovirus* are cleaned using the food cleaning treatment agent that is the food and drink composition of the present invention is preferable, and it is sufficient to add the treatment agent to water for rinsing raw oysters at the time of cleaning or to immerse raw oysters in the water containing the treatment agent.

The preferable ingestion amount of the food and drink composition of the present invention is not particularly limited as long as the antiviral effect can be exhibited, and for example, when an average human having the body weight of about 60 kg is a target, the amount is 0.01 mg to 10,000 mg, preferably 0.1 mg to 10,000 mg, and more preferably 1 mg to 5,000 mg as the amount of the serine protease per day, and is 0.01 mg to 100,000 mg, preferably 0.1 mg to 100,000 mg, and more preferably 1 mg to 50,000 mg as the amount of the cationic polymer per day.

The preferable ingestion timing of the food and drink composition of the present invention is not particularly limited, and may be any of before meals, after meals, and between meals, for example. The ingestion interval is also not particularly limited.

The content of the antiviral agent of the present invention in the food and drink composition of the present invention is not particularly limited as long as the antiviral activity is exerted, and may be arbitrary.

Still another aspect of the present invention is use of the serine protease and the cationic polymer in the manufacture of the food and drink composition.

Still another aspect is a method for ameliorating and/or preventing viral infectious diseases, the method including causing to ingest the serine protease and the cationic polymer, or the food and drink composition containing them as active ingredients.

### <Aspects of other products>

In addition, the antiviral agent of the present invention can be in various forms such as daily necessities and hygiene products.

The form in this case is not particularly limited, but the form of an aerosolized agent can be used. The aerosolized agent is used in the form of sprays, aerosols, or aerosolization drying, for example. The aerosolized agent is processed into a dosage form suitable for aerosolization and/or ejection by compressed gas or pump. For example, the antiviral agent of the present invention is dissolved in water, a buffer having a pH of 6.0 or higher, preferably about a pH of 7.0 to 11.0, and more preferably about a pH of 7.3 to 10.3 although there is no particular limitation, physiological saline, or the like, or is suspended in a volatile solvent to be formulated. The aerosolized agent can be directly administered to a biological body in the form of an aerosolized agent for oral cavity, an aerosolized agent for nasal cavity, or the like, or can be used by being applied to textile products such as face masks and clothes, or by being dispersed to the inside of rooms in the form of a humidifier or the like.

In addition, the present invention also includes a textile product obtained by applying the antiviral agent of the present invention.

The textile product is clothing made from synthetic fibers, regenerated fibers, or natural fibers, or blended fabric thereof, hygiene products, bedding, films, or interior goods. Examples of the textile product include, but are not limited to, shirts, coats, trousers, skirts, nightwear, underwear, socks, gloves, hats, white gowns, nurse gowns, home nursing care clothes, work clothes, face masks, sheets, pillow covers, carpets, bedclothes, seat cushions, wallpaper, various filters, curtains, and carpets.

Regarding the textile product according to the present invention, it is sufficient to apply the antiviral agent of the present invention to a textile product or fibers before product processing by a method such as spraying, immersion, impregnation, or coating, for example.

When the antiviral agent of the present invention is in the form of various products (including the food and drink composition), the antiviral agent can be optionally used in combination with appropriate ingredients as long as the effect of the present invention is not impaired.

Such ingredients for combination use may be appropriately selected according to the dosage form and the like of a product, and examples thereof include, but are not limited to, water; lower alcohols having 5 or less carbon atoms such as ethanol and isopropanol; polyhydric alcohols such as glycerin, polyethylene glycol, butylene glycol, propylene glycol, dipropylene glycol, and sorbitol; and antibacterial agents such as glycine, organic acids, glycerin fatty acid esters, sucrose fatty acid esters, sodium benzoate, sodium sorbate, sodium propionate, sodium dehydroacetate, p-hydroxybenzoate ester, sodium sulfite, EDTA, benzalkonium chloride, benzethonium chloride, chlorhexidine gluconate, alkyldiaminoethylglycine chloride, iodine tincture, povidone iodine, cetyl benzalkonium oxide, triclosan, chloroxylenol, isopropyl methylphenol, lactoferrin, nisin, bacteriocin, *Aralia cordata* extract, *Styrax japonica* extract, *Artemisia capillaris* extract, enzymatically degraded *Coix lacryma-jobi* extract, fish milt protein extract, thujaplicin, and pectin decomposition product. Among these, as the ingredient for combination use, at least one selected from the group consisting of water, lower alcohols, and polyhydric alcohols is preferable, and from the viewpoint of imparting antibacterial properties, at least one selected from the group consisting of lower alcohols and polyhydric alcohols is more preferable.

In addition, preferable examples of the ingredients for combination use also include additives such as pH adjusters such as citric acid, sodium citrate, sodium hydroxide, and triethanolamine; antioxidants such as tocopherol acetate; thickeners such as carboxyvinyl polymers and hydroxyethyl cellulose; aminocarboxylic acid-based chelating agents such as ethylenediaminetetraacetic acid, nitrilotriacetic acid, diethylenetriaminepentaacetic acid, hydroxyethylethylenediaminetriacetic acid, triethylenetetraminehexaacetic acid, and cyclohexanediaminetetraacetic acid; phosphonic acid-based chelating agents such as hydroxyethylidene diphosphonic acid, nitrilotris(methylenephosphonic acid), phosphonobutanetricarboxylic acid, and ethylenediaminetetramethylenephosphonic acid; and amine-based chelating agents such as triethanolamine and polyamines.

### [Examples]

The present invention will be described in more detail below with reference to examples, but it is needless to say that the scope of the present invention is not limited merely to the examples.

### <Example 1> Anti-Feline calicivirus activity test when combination use of subtilisin or nattokinase with ε-polylysine (evaluation by plaque assay method)

A *Feline calicivirus* F-9 ATCC VR-782 was diluted to a predetermined concentration with respect to cells. The diluted virus solution was added to CRFK cells (ATCC CCL-94) having the surface washed with phosphate buffered saline (PBS (-) (pH 7.4)). 5 mL of a cell growth culture medium (Dulbecco's Modified Eagle Medium supplemented with 2% fetal bovine serum, Nacalai Tesque Inc.) was added to cause culture in a CO₂ incubator for 2 or 3 days under the culture conditions of 37°C and 5% CO₂ until a cytopathic effect (CPE) was confirmed. The suspension was recovered in a 50 mL conical tube and centrifuged (at 3,000 rpm for 10 minutes) to make the cells into pellets. The supernatant solution obtained by centrifugation was dispensed into glass vials to obtain virus stock solutions, which were stored at -85°C.

Subtilisin (Sigma-Aldrich, product number P4860, enzyme activity ≥ 2.4 U/g, composition (weight/volume percent): enzyme 9%; glycerol 50%; water 41%), nattokinase (FUJIFILM Wako Pure Chemical Corporation, product number 147-08801, activity ≥ 250 IU/g, molecular weight of about 31,000), and ε-polylysine (JNC Corporation, lot number 2161102, weight-average molecular weight 4700, weight/volume percent concentration 25%) were respectively prepared with purified water such that the concentration was a predetermined final concentration (volume percent concentration) of each test plot in tables.

After 0.05 mL of the virus stock solution was added to 0.45 mL of a sample and left to stand at room temperature for 30 minutes, the mixture was diluted 10-fold with a cell growth culture medium (Dulbecco's Modified Eagle Medium supplemented with 10% fetal bovine serum, Nacalai Tesque Inc.), and thereafter similarly serially diluted from 10²-fold to 10⁶-fold with the cell growth culture medium. The liquid obtained by this serial dilution was used as a serially diluted solution.

Using the cell growth culture medium, the infected cells (CRFK cells) were preliminarily seeded in a 12-well plate for tissue culture (TrueLine cell culture plate TR5001) at the cell number of 4 × 10⁵ cells per well, and cultured in a CO₂ incubator for 1 day under the culture conditions of 37°C and 5% CO₂.

The cells of each well were inoculated with 0.3 mL of the serially diluted solution, cultured again in a CO₂ incubator for 1 hour under the culture conditions of 37°C and 5% CO₂, and washed twice with 1 mL of the cell growth culture medium to be overlaid thereafter with a culture medium for layering (Dulbecco's Modified Eagle Medium supplemented with 1% CMC and 2% fetal bovine serum, Nacalai Tesque Inc.).

After culturing in a CO₂ incubator for 2 days under the culture conditions of 37°C and 5% CO₂, immobilization and staining with a gentian violet (crystal violet) solution were performed to measure the number of plaques. The number of plaques measured was applied to the equation ((number of plaques per well/volume (mL) of specimen to be measured per well) × dilution factor = PFU/mL) to calculate the virus titer (PFU/mL).

As shown in Table 1, it was found that the case (Sample #3) of using subtilisin and ε-polylysine in combination resulted in a greater decrease in the virus titer than the case (Sample #2) of using subtilisin alone. Furthermore, as shown in Table 2, it was found that the case (Sample #6) of using nattokinase and ε-polylysine in combination resulted in a greater decrease in the virus titer than the case (Sample #5) of using nattokinase alone.

In other words, it was shown that the combination use of subtilisin or nattokinase and ε-polylysine greatly enhanced the antiviral effect as compared to the case of using subtilisin or nattokinase alone.

### [Table 1]

**Table 1**

| Sample | ε-Polylysine | Subtilisin | PFU/mL |
|---|---|---|---|
| #1 | - | - | 4.66 × 10⁵ |
| #2 | - | 40 ppm | 8.66 × 10¹ |
| #3 | 0.1% | 40 ppm | 1.33 × 10¹ |

### [Table 2]

**Table 2**

| Sample | ε-Polylysine | Nattokinase | PFU/mL |
|---|---|---|---|
| #4 | - | - | 3.33 × 10⁵ |
| #5 | - | 0.1% | 1.33 × 10² |
| #6 | 0.1% | 0.1% | 5.99 × 10¹ |

### <Example 2> Anti-Feline calicivirus activity test when combination use of subtilisin or nattokinase with ε-polylysine (evaluation with log TCID₅₀ value)

It was confirmed whether the *anti-Feline calicivirus* activity was enhanced by the combination use of subtilisin or nattokinase with ε-polylysine.

This test was conducted multiple times, and samples were respectively prepared at the mixed concentrations shown in Table 3 and Table 4.

### [Table 3]

**Table 3**

| Test content (first time) | ε-Polylysine | Serine protease |
|---|---|---|
| Negative control | 0 | 0 |
| Subtilisin (ε-polylysiue-) | 0 | 1000 ppm |
| Subtilisin (ε-polylysine+) | 1000 ppm | 1000 ppm |

### [Table 4]

**Table 4**

| Test content (second time) | ε-Polylysine | Serine protease |
|---|---|---|
| Negative control | 0 | 0 |
| ε-Polylysine | 1000 ppm | 0 |
| Subtilisin (ε-polylysine-) | 0 | 20 ppm |
| Subtilisin (ε-polylysine+) | 1000 ppm | 20 ppm |
| Nattokinase (100 ppm, ε-polylysine-) | 0 | 100 ppm |
| Nattokinase (100 ppm, ε-polylysine+) | 1000 ppm | 100 ppm |
| Nattokinase (1000 ppm, ε-potylysine-) | 0 | 1000 ppm |
| Nattokinase (1000 ppm, ε-polylsine+) | 1000 ppm | 1000 ppm |

The virus stock solution of the *Feline calicivirus* solution used in Example 1 was appropriately diluted with PBS (-) (pH 7.4) such that the dilution ratio was 10 times or less and the log TCID₅₀ value of a negative control was not less than 6, and the virus stock solution and a serine protease solution were mixed at the volume ratio of 1:9 and incubated for 30 minutes at room temperature. After incubation, the culture supernatant was diluted with PBS (-) (pH 7.4) to create 10⁰-fold to 10⁹-fold serially diluted solutions.

Using a cell growth culture medium (Eagle's Minimum Essential Medium supplemented with 5% fetal bovine serum, Sigma-Aldrich), the infected cells (CRFK cells ATCC CCL-94) were preliminarily seeded in a 96-well plate for tissue culture (IWAKI microplate for cell culture, made of polystyrene, flat bottom) at the cell number of 2 × 10⁴ cells per well (0.05 mL of cell growth culture medium), and cultured in a CO₂ incubator under the culture conditions of 37°C and 5% CO₂. After incubation, 0.05 mL of the serially diluted solution was added per well and mixed without removing the culture medium from each well to inoculate the cells with the virus solution. The cells were cultured in a CO₂ incubator for 2 or 3 days under the culture conditions of 37°C and 5% CO₂, and the cytopathicity was confirmed by with a microscope to measure the log TCID₅₀ value by a method known to those skilled in the art.

The log TCID₅₀ value was decreased by 1.4 to 2.3 by the combination use of subtilisin and ε-polylysine, as compared to the use of subtilisin alone (Table 5 and Table 6). In addition, the log TCID₅₀ value was decreased by 3.0 at the maximum (100 ppm of nattokinase) by the combination use of nattokinase and ε-polylysine, as compared to the use of nattokinase alone (Table 6). In other words, it was shown that the combination use of subtilisin or nattokinase and ε-polylysine synergistically increased the antiviral activity as compared to the case of using each of subtilisin, nattokinase, or ε-polylysine alone.

### [Table 5]

**Table 5**

| Test content (first time) | logTCID₅₀/mL | Antiviral activity value |
|---|---|---|
| Negative control | 7.2 | - |
| Subtilisin (ε-polylysine-) | 4.3 | 2.9 |
| Subtilisin (ε-polylysine+) | 2.9 | 4.3 |

### [Table 6]

**Table 6**

| Test content (second time) | logTCID₅₀/mL | Antiviral activity value |
|---|---|---|
| Negative control | 6.8 | - |
| ε-Polylysine | 5.9 | 0.9 |
| Subtilisin (ε-polylysine-) | 5.1 | 1.7 |
| Subtilisin (ε-polylsine+) | 2.8 | 4.0 |
| Nattokinase (100 ppm, ε-polylysine-) | 6.1 | 0.7 |
| Nattokinase (100 ppm, ε-polylysine+) | 3.1 | 3.7 |
| Nattokinase (1000 ppm, ε-polylysine-) | 5.3 | 1.5 |
| Nattokinase (1000 ppm, ε-polylysine+) | 2.8 | 4.0 |

### <Example 2> Anti-Feline calicivirus activity test when combination use of commercially available enzyme formulation containing serine protease with ε-polylysine (evaluation with log TCID₅₀ value)

It was confirmed whether the *anti-Feline calicivirus* activity was enhanced by the combination use of a commercially available enzyme formulation containing a serine protease with ε-polylysine. Enzyme formulations shown in Tables 7 and 8 were used and adjusted with PBS (-) (pH 7.4) such that the concentration was a predetermined final concentration (volume percent concentration) of each test plot in the tables to prepare serine protease solutions.

This test was conducted multiple times, and samples were respectively prepared at the mixed concentrations shown in Table 7 and Table 8.

### [Table 7]

**Table 7**

| Test content | | Formulation concentration | ε-Polylysine |
|---|---|---|---|
| Negative control | - | 0 | - |
| Protin SD-AY10 (ε-polylysine-) | Manufactured by Amano Enzyme Inc. | 250 ppm | - |
| Protin SD-AY10 (ε-polylysine+) | Manufactured by Amano Enzyme Inc. | 250 ppm | 0.10% |
| Bioprase (registered trademark) OP (ε-polylysine-) | Manufactured by Nagase ChemteX Corporation | 250 ppm | - |
| Bioprase (registered trademark) OP (ε-polylysine+) | Manufactured by Nagase ChemteX Corporation | 250 ppm | 0.10% |
| Bioprase (registered trademark) SP-20FG (ε-polylysine-) | Manufactured by Nagase ChemteX Corporation | 250 ppm | - |
| Bioprase (registered trademark) SP-20FG (ε-polylysine+) | Manufactured by Nagase ChemteX Corporation | 250 ppm | 0.10% |

### [Table 8]

**Table 8**

| Test content | | Formulation concentration | ε-Polylysine |
|---|---|---|---|
| Negative control | - | 0 | - |
| Alcalase (registered trademark) 2.4 L FG (ε-polylysine-) | Manufactured by Novozymes Japan Ltd. | 250 ppm | - |
| Alcalase (registered trademark) 2.4 L FG (ε-polylysine+) | Manufactured by Novozymes Japan Ltd. | 250 ppm | 0.10% |

The virus stock solution of the *Feline calicivirus* solution used in Example 1 was appropriately diluted with PBS (-) (pH 7.4) such that the dilution ratio was 10 times or less and the log TCID₅₀ value of a negative control was not less than 6, and the virus stock solution and a serine protease solution were mixed at the volume ratio of 1:9 and incubated for 10 minutes at room temperature. After incubation, the culture supernatant was diluted with PBS (-) (pH 7.4) to create 10⁰-fold to 10⁹-fold serially diluted solutions.

Using a cell growth culture medium (Eagle's Minimum Essential Medium supplemented with 5% fetal bovine serum, Sigma-Aldrich), the infected cells (CRFK cells ATCC CCL-94) were preliminarily seeded in a 96-well plate for tissue culture (IWAKI microplate for cell culture, made of polystyrene, flat bottom) at the cell number of 2 × 10⁴ cells per well (0.05 mL of cell growth culture medium), and cultured in a CO₂ incubator under the culture conditions of 37°C and 5% CO₂. After incubation, 0.05 mL of the serially diluted solution was added per well and mixed without removing the culture medium from each well to inoculate the cells with the virus solution. The cells were cultured in a CO₂ incubator for 2 or 3 days under the culture conditions of 37°C and 5% CO₂, and the cytopathicity was confirmed by with a microscope to measure the log TCID₅₀ value by a method known to those skilled in the art.

When the commercially available enzyme formulation containing a serine protease was used in combination with ε-polylysine, the log TCID₅₀ value decreased by 3.1 to 4.5 as compared to negative controls (Table 9 and Table 10). In other words, by the combination use of the commercially available enzyme formulation and ε-polylysine, it was shown that the antiviral activity was synergistically increased and was shown that the antiviral effect thereof was stably expressed, as compared to the case of using each of the commercially available enzyme formulations alone.

### [Table 9]

**Table 9**

| Test content | logTCID₅₀/mL | Antiviral activity value |
|---|---|---|
| Negative control | 6.4 | - |
| Protin SD-AY10 (ε-polylysine-) | 6.3 | 0.1 |
| Protin SD-AY10 (ε-polylysine+) | 3.3 | 3.1 |
| Bioprase (registered trademark) OP (ε-polylysine-) | 6.3 | 0.1 |
| Bioprase (registered trademark) or (ε-polylysine+) | 2.8 | 3.6 |
| Bioprase (registered trademark) SP-20FG (ε-polylysine-) | 6.3 | 0.1 |
| Bioprase (registered trademark) SP-20FG (ε-polylysine+) | 2.8 | 3.6 |

### [Table 10]

**Table 10**

| Test content | logTCID₅₀/mL | Antiviral activity value |
|---|---|---|
| Negative control | 7.4 | - |
| Alcalase (registered trademark) 2.4 L FG (ε-polylysine-) | 7.2 | 0.2 |
| Alcalase (registered trademark) 2.4 L FG (ε-polylysine+) | 2.9 | 4.5 |

### <Example 4> Anti-Feline calicivirus activity test when combination use of subtilisin with several types of polycations (evaluation with log TCID₅₀ value)

It was confirmed whether the *anti-Feline calicivirus* activity was enhanced by the combination use of subtilisin with several types of polycations. Subtilisin used in Example 1, polyethylenimine (JUNSEI CHEMICAL CO., LTD., product number: 2018I1645, molecular weight of about 1200), protamine sulfate (FUJIFILM Wako Pure Chemical Corporation, product number: 168-05192, derived from salmon), and DEAE-Dextran hydrochloride (Sigma-Aldrich, product number: 29100346) were respectively used to prepare samples at the mixed concentrations shown in Table 11. A sample #1 is a negative control.

0.45 mL of the sample and 0.05 mL of the virus stock solution of the *Feline calicivirus* solution used in Example 1 were mixed and incubated at room temperature for 30 minutes. After incubation, the culture supernatant was diluted with PBS (-) (pH 7.4) to create 10⁰-fold to 10⁹-fold serially diluted solutions.

Using a cell growth culture medium (Eagle's Minimum Essential Medium supplemented with 5% fetal bovine serum, Sigma-Aldrich), the infected cells (CRFK cells ATCC CCL-94) were preliminarily seeded in a 96-well plate for tissue culture (IWAKI microplate for cell culture, made of polystyrene, flat bottom) at the cell number of 2 × 10⁴ cells per well, and cultured in a CO₂ incubator under the culture conditions of 37°C and 5% CO₂. The cells of each well were inoculated with 0.05 mL of the serially diluted solution. The cells were cultured in a CO₂ incubator for 2 or 3 days under the culture conditions of 37°C and 5% CO₂. and the cytopathicity was confirmed by with a microscope to measure the log TCID₅₀ value by a method known to those skilled in the art.

As shown in Table 11, the log TCID₅₀ value in the case of combination use of subtilisin and polyethylenimine was 3.7, which was a decrease by 2.4 as compared to the negative control (6.1), showing that the combination use of subtilisin and polyethylenimine exerted the antiviral activity. On the other hand, the log TCID₅₀ values in the case of combination use of subtilisin with protamine sulfate or DEAE-Dextran hydrochloride were respectively 6.6 and 5.7, which were values similar to that of the negative control, showing that the antiviral activity was not exerted even by the combination use of subtilisin with protamine sulfate or DEAE-Dextran hydrochloride.

### [Table 11]

**Table 11**

| Sample | Subtilisin | Polyethylenimine | Protamine sulfate | DEAE-Dextran hydrochloride | logTCID₅₀/mL |
|---|---|---|---|---|---|
| #1 | 0 | 0 | 0 | 0 | 6.1 |
| #2 | 20 ppm | 0.1% | 0 | 0 | 3.7 |
| #3 | 20 ppm | 0 | 0.1% | 0 | 6.6 |
| #4 | 20 ppm | 0 | 0 | 0.1% | 5.7 |

### <Example 5> Anti-Feline calicivirus activity test when combination use of subtilisin or nattokinase with ε-polylysine in seawater (evaluation with log TCID₅₀ value)

It was confirmed whether the *anti-Feline calicivirus* activity was exerted when subtilisin or nattokinase was used in combination with ε-polylysine in seawater.

The seawater used was the following artificial seawater A and B.

Artificial seawater A: preparation was made by putting one bag (about 18.5g) of artificial seawater for ornamental fish (KAMIHATA FISH INDUSTRIES LTD., no product number) in 500 mL of tap water according to the use method described in the manufacturer's manual. Furthermore, autoclaving treatment was performed for sterilization.

Artificial seawater B: a Tetra Marine Salt Pro (Tetra, product number: 70751) was adjusted to be 3.5% by weight with deionized water, and autoclaving treatment was performed for sterilization.

Samples were prepared at the mixed concentrations shown in Table 12 to obtain serine protease solutions. A dilution solvent was PBS (-) (pH 7.4) for the samples #1 to #3, and was artificial seawater A or artificial seawater B for the samples #4 to #7.

The virus stock solution of the *Feline calicivirus* solution used in Example 1 and the serine protease solution were mixed at the volume ratio of 1:9 and incubated at room temperature for 30 minutes. After incubation, the culture supernatant was diluted with PBS (-) (pH 7.4) to create 10⁰-fold to 10⁹-fold serially diluted solutions.

Using a cell growth culture medium (Eagle's Minimum Essential Medium supplemented with 5% fetal bovine serum, Sigma-Aldrich), the infected cells (CRFK cells ATCC CCL-94) were preliminarily seeded in a 96-well plate for tissue culture (IWAKI microplate for cell culture, made of polystyrene, flat bottom) at the cell number of 2 × 10⁴ cells per well (0.05 mL of cell growth culture medium), and cultured in a CO₂ incubator under the culture conditions of 37°C and 5% CO₂. After incubation, 0.05 mL of the serially diluted solution was added per well and mixed without removing the culture medium from each well to inoculate the cells with the virus solution. The cells were cultured in a CO₂ incubator for 2 or 3 days under the culture conditions of 37°C and 5% CO₂, and the cytopathicity was confirmed by with a microscope to measure the log TCID₅₀ value by a method known to those skilled in the art.

### [Table 12]

**Table 12**

| Sample | Solvent | ε-Polylysine | Subtilisin | Nattokinase | logTCID₅₀/mL |
|---|---|---|---|---|---|
| #1 | PBS (-) | 0 | 0 | 0 | 6.2 |
| #2 | PBS (-) | 0.1% | 20 ppm | 0 | 3.7 |
| #3 | PBS (-) | 0.1% | 0 | 0.1% | 3.2 |
| #4 | Artificial seawater A | 0.1% | 20 ppm | 0 | 3.9 |
| #5 | Artificial seawater B | 0.1% | 20 ppm | 0 | 3.9 |
| #6 | Artificial seawater A | 0.1% | 0 | 0.1% | 5.1 |
| #7 | Artificial seawater B | 0.1% | 0 | 0.1% | 4.3 |

As shown in Table 12, in the case of using nattokinase and ε-polylysine in combination, it was confirmed that the *anti-Feline calicivirus* activity of the samples (#6 to #7) for which the artificial seawater A or B was used was exerted as compared to the control sample (#1), although the antiviral activity of the samples (#6 to #7) was slightly inferior as compared to the sample (#3) for which the PBS (-) (pH 7.4) was used.

In addition, in the case of using subtilisin and ε-polylysine in combination, the *anti-Feline calicivirus* activity of the samples (#4 and #5) for which the artificial seawater A or B was used was equivalent in terms of the log TCID₅₀ value as compared to the sample (#2) for which the PBS (-) (pH 7.4) was used, showing that a high antiviral activity was maintained even in seawater.

### <Comparative Example 1> Anti-Feline calicivirus activity test when combination use of natto extract with ε-polylysine in seawater (evaluation with log TCID₅₀ value)

300 g of natto (Yamada Foods., micro-grain natto for commercial use YM-180) was weighed, 10 mM Tris-HCl (pH 7.4) was added in the amount 3 times the amount of the natto, and the mixture was stirred overnight in a refrigerator. The natto was removed with gauze, and the solution thereof was centrifuged at 5,000 × g for 10 minutes to remove the precipitate. ε-Polylysine (JNC Corporation, 25% polylysine aqueous solution 2161102) was added to such that the concentration was 0.1%, and mixed with a stirrer for 1 hour, and thereafter centrifugation was performed at 5,000 × g for 10 minutes to remove the precipitate. Filtration and sterilization were performed using a 0.22 µm sterilized filter (TRP Syringe filters 0.22 µm). Ammonium sulfate (FUJIFILM Wako Pure Chemical Corporation, reagent special grade 019-03435) was added in several portions such that the saturation concentration was 50%, and the mixture was stirred at 4°C for 1 hour. This solution was centrifuged at 10,000 × g for 20 minutes to remove the precipitate, ammonium sulfate was added to the supernatant in several portions such that the saturation concentration was 75%, and the mixture was stirred at 4°C for 1 hour. After centrifugation at 10,000 × g for 20 minutes, the supernatant was discarded, and 10 mL of 10 mM Tris-HCl was added to the precipitate under ice-cooling and dissolved to obtain a solution to be dialyzed. About 25 cm of a dialysis membrane (SEKISUI MEDICAL CO., LTD., cellulose tube dialysis membrane 27/32, manufacturing number: 521737) was washed with distilled water to remove a plasticizer, and the above-mentioned solution to be dialyzed was enclosed in the dialysis membrane together with air. 10 mM Tris-HCl having the volume approximately 100 times that of the solution to be dialyzed was used as an external liquid, and the dialysis membrane enclosing the solution to be dialyzed was floated thereon to cause dialysis overnight while stirring the external liquid. At that time, the external liquid was exchanged about three times. The dialysis membrane was cut open to transfer the solution after dialysis to a container. The mouth of this container was covered with a KimWipes and a rubber band instead of a lid, and freezing was performed in a -85°C deep freezer for about 1 hour. This container was attached to a freeze dryer (TOKYO RIKAKIKAI CO., LTD., EYELA FDU-2000), and freeze-drying was performed for about two days and nights to obtain a natto extract. The natto extract thus obtained was dissolved in sterilized water such that the concentration was 0.2%, and filtered and sterilized with a 0.22 µm sterilized filter.

Artificial seawater A and artificial seawater B which were the same as those used in Example 4 were used.

Samples were prepared at the mixed concentrations shown in Tables 13 to 15. As solvents, sterilized water was used for test plots #1 and #2, the artificial seawater A was used for test plots #3 and 4, and the artificial seawater B was used for test plots #5 and #6 to obtain sample solutions. The virus stock solution and the sample solution were mixed at the volume ratio of 1:9, and incubated for 30 minutes at room temperature. After incubation, the culture supernatant was diluted with PBS (-) (pH 7.4) to create 10⁰-fold to 10⁹-fold serially diluted solutions.

The log TCID₅₀ value was measured by a method similar to that described in Example 5. As a result, when the natto extract and polylysine were used in combination, the log TCID₅₀ value was 4.5 in the case of the sterilized water, which was a decrease by 2.7 as compared to a negative control (7.2), showing that the *anti-Feline calicivirus* activity was exerted (Table 13). On the other hand, in the case of the artificial seawater A and B, the log TCID₅₀ values were respectively 6.3 and 6.6, showing that a decrease stopped at 0.9 and 0.7 as compared to negative controls (7.2 and 7.3) (Table 14 and Table 15). In other words, it was shown that the *anti-Feline calicivirus* activity when the natto extract and polylysine were used in combination in seawater was significantly reduced as compared to when they were used in combination in sterilized water.

### [Table 13]

**Table 13**

| Test plot | Solvent | ε-Polylysine | Natto extract | logTCID₅₀/mL |
|---|---|---|---|---|
| #1 | Sterilized water | 0 | 0 | 7.2 |
| #2 | Sterilized water | 0.025% | 0.025% | 4.5 |

### [Table 14]

**Table 14**

| Test plot | Solvent | ε-Polylysine | Natto extract | logTCID₅₀/mL |
|---|---|---|---|---|
| #3 | Artificial seawater A | | 0 | 7.2 |
| #4 | Artificial seawater A | 0.1% | 0.1% | 6.3 |

### [Table 15]

**Table 15**

| Test plot | Solvent | ε-Polylysine | Natto extract | logTCID₅₀/mL |
|---|---|---|---|---|
| #5 | Artificial seawater B | 0 | 0 | 7.3 |
| #6 | Artificial seawater B | 0.1% | 0.1% | 6.6 |

### <Example 6> Anti-Feline calicivirus activity test when combination use of trypsin and ε-polylysine (evaluation with log TCID₅₀ value)

It was examined whether the *anti-Feline calicivirus* activity was enhanced by using trypsin in combination with ε-polylysine. Trypsin derived from porcine spleen (FUJIFILM Wako Pure Chemical Corporation, product number 209-19282, lot number SEG1875) and ε-polylysine (JNC Corporation, weight-average molecular weight 4700, weight/volume percent concentration 25%, lot number 2161102) were prepared with Tris-HCl such that the concentration was a predetermined final concentration (volume percent concentration) of each test plot in Table 16.

The virus stock solution of the *Feline calicivirus* solution used in Example 1 was appropriately diluted with PBS (-) (pH 7.4) such that the dilution ratio was 10 times or less and the log TCID₅₀ value of a negative control was not less than 6, and the virus stock solution and a serine protease solution were mixed at the volume ratio of 1:9 and incubated for 30 minutes at room temperature. After incubation, the culture supernatant was diluted with PBS (-) (pH 7.4) to create 10⁰-fold to 10⁹-fold serially diluted solutions.

Using a cell growth culture medium (Eagle's Minimum Essential Medium supplemented with 5% fetal bovine serum, Sigma-Aldrich), the infected cells (CRFK cells ATCC CCL-94) were preliminarily seeded in a 96-well plate for tissue culture (IWAKI microplate for cell culture, made of polystyrene, flat bottom) at the cell number of 2 × 10⁴ cells per well (0.05 mL of cell growth culture medium), and cultured in a CO₂ incubator under the culture conditions of 37°C and 5% CO₂. After incubation, 0.05 mL of the serially diluted solution was added per well and mixed without removing the culture medium from each well to inoculate the cells with the virus solution. The cells were cultured in a CO₂ incubator for 2 or 3 days under the culture conditions of 37°C and 5% CO₂, and the cytopathicity was confirmed by with a microscope to measure the log TCID₅₀ value by a method known to those skilled in the art.

As shown in Table 16, when trypsin was used alone at 500 ppm which was a low concentration, the antiviral activity was not exerted, whereas when trypsin was used in combination with e-polylysine, the log TCID₅₀ value was 4.9, which was a decrease by 1.9 as compared to a negative control (6.8), showing that the combination use of trypsin and ε-polylysine can enhance the antiviral activity.

### [Table 16]

**Table 16**

| Test content | ε-Polylysine | Trypsin | logTCID₅₀/mL | Antiviral activity |
|---|---|---|---|---|
| Negative control | 0 | 0 | 6.8 | - |
| Trypsin (ε-polylysine-) | 0 | 500 ppm | 6.8 | 0.0 |
| Trypsin (ε-polylysine+) | 1000 ppm | 500 ppm | 4.9 | 1.9 |

### <Example 7> Cytotoxicity test

Subtilisin, nattokinase, and ε-polylysine used in Example 1 were appropriately diluted with a Dulbecco's Modified Eagle Medium supplemented with 10% fetal bovine serum such that the concentration of each of them was 0.5 to 5,000 µg/mL, and were added to the culture supernatant of RAW 264.7 cells cultured in a 48-well plate. The cell numbers were measured after 72 hours to obtain 50% cytotoxic concentration (CC₅₀) by a method known to those skilled in the art.

The CC₅₀ when using the value obtained by averaging two measurement values of the cell numbers was 4.6 µg/mL for subtilisin, 1,450 µg/mL for nattokinase, and 27 µg/mL for ε-polylysine.

### <Example 8> Anti-Murine norovirus activity test

Each of subtilisin, nattokinase, and ε-polylysine used in Example 1 was diluted with PBS (-) (pH 7.4) to prepare a diluted solution of 4-fold at the final concentration.

A stock solution of a *Murine norovirus* was prepared by the following method. 1 × 10⁶ cells/mL of the RAW 264.7 cells were added to 100 mm dishes (10 mL per dish) and cultured in a CO₂ incubator under the culture conditions of 37°C and 5% CO₂. A DMEM supplemented with 10% fetal bovine serum was used as a culture medium. On the next day, a virus solution of a *Murine norovirus* (MNV S7-PP3 strain) was prepared at 0.1 PFU/cells, and 1 mL thereof was added to the above-mentioned cells of each dish to infect the cells at room temperature for 1 hour. 5 mL of a maintenance culture medium (DMEM supplemented with 1% fetal bovine serum) was added to each dish to culture the cells in a CO₂ incubator at the culture conditions of 37°C and 5% CO₂. On the next day, after confirming that CPE (cytopathic effect due to virus infection) appeared in the entire cells, the suspension was put in a 50 mL tube and centrifuged at 4°C and 1,500 rpm for 10 minutes. This supernatant was appropriately dispensed (0.1 to 1 mL) in 1.5 mL microtubes to obtain virus stock solutions, which were stored at -80°C.

The virus stock solution was prepared by being diluted with PBS (-) (pH 7.4) such that the virus titer was 2 × 10⁵ PFU/mL.

20 µL of the subtilisin or nattokinase prepare above or PBS (-) (pH 7.4), 20 µL of the ε-polylysine prepared above or PBS (-) (pH 7.4), and 40 µL of the virus stock solution prepared above (80 µL/wells in total) were added to each well of a 96-well plate, and left to stand at room temperature for 30 minutes to 1 hour. Thereafter, 10 µL was removed from each well to be immediately diluted 100-fold with PBS (-) (pH 7.4), and 100 µL was recovered. The RAW 264.7 cells cultured in a monolayer form in a 24-well plate were inoculated with this diluted solution, and overlaid with a culture medium for plaques. The cells were immobilized 2 days after the culture and stained with a gentian violet (crystal violet) solution by a method known to those skilled in the art, and thereafter the number of plaques were measured. As shown in Table 17, it was found that, in the case of using subtilisin or nattokinase in combination with ε-polylysine, the number of plaques was smaller as compared to the case of using subtilisin, nattokinase, or ε-polylysine alone.

In other words, it was shown that the combination use of subtilisin or nattokinase and ε-polylysine greatly enhanced the antiviral effect as compared to the case of using subtilisin or nattokinase alone.

### [Table 17]

**Table 17**

| Subtilisin (µg/mL) | Nattokinase (µg/mL) | ε-Polylysine (µg/mL) | Number of plaques | |
|---|---|---|---|---|
| | | | 30 min | 1 h |
| - | - | - | 27 | 14 |
| 1000 | - | - | 45 | 2 |
| 500 | - | - | 46 | 0 |
| 100 | - | - | 48 | 0 |
| 20 | - | - | 54 | 13 |
| 1000 | - | 1000 | 12 | 6 |
| 500 | - | 1000 | 1 | 0 |
| 100 | - | 1000 | 0 | 0 |
| 20 | - | 1000 | 3 | 0 |
| 1000 | - | 500 | 0 | 0 |
| 500 | - | 500 | 0 | 0 |
| 100 | - | 500 | 0 | 0 |
| 20 | - | 500 | 1 | 0 |
| 1000 | - | 200 | 0 | 0 |
| 500 | - | 200 | 0 | 0 |
| 100 | - | 200 | 7 | 0 |
| 20 | - | 200 | 2 | 0 |
| - | 1000 | - | 48 | 37 |
| - | 500 | - | 44 | 36 |
| - | 100 | - | 29 | 9 |
| - | 20 | - | 27 | 9 |
| - | 1000 | 1000 | 9 | 0 |
| - | 500 | 1000 | 5 | 0 |
| - | 100 | 1000 | 2 | 0 |
| - | 20 | 1000 | 5 | 0 |
| - | 1000 | 500 | 17 | 7 |
| - | 500 | 500 | 6 | 1 |
| - | 100 | 500 | 4 | 2 |
| - | 20 | 500 | 3 | 4 |
| - | 1000 | 200 | 15 | 5 |
| - | 500 | 200 | 11 | 6 |
| - | 100 | 200 | 14 | 7 |
| - | 20 | 200 | 12 | 4 |
| - | - | 1000 | 10 | 0 |
| - | - | 500 | 10 | 1 |
| - | - | 200 | 16 | 2 |

### <Example 9> Anti-Murine norovirus activity test when combination use of nattokinase and ε-polylysine with alcohol (evaluation by plaque assay method)

A *Murine norovirus* same as that used in Example 8 was used.

Nattokinase (FUJIFILM Wako Pure Chemical Corporation, product number 147-08801, activity ≥ 250 IU/g, molecular weight of about 31,000) and ε-polylysine (JNC Corporation, lot number 2161102, weight-average molecular weight 4700, weight/volume percent concentration 25%) were diluted with PBS (-) (pH 7.4) to adjust the concentration of each thereof to 1% (w/v), and 0.01 mL thereof was mixed in each well of a 96-well plate. 0 or 0.05 mL of 99.5% ethanol (FUJIFILM Wako Pure Chemical Corporation, product number 057-00456) was added thereto, and PBS (-) (pH 7.4) is added such that the total liquid volume was 0.075 mL. In addition, 0.075 mL of PBS (-) (pH 7.4) was prepared as a negative control. 0.025 mL of the virus stock solution was added to these wells, left to stand at room temperature for 1 hour, and thereafter diluted 100-fold with PBS (-) (pH 7.4).

Using the cell growth culture medium (DMEM supplemented with 10% fetal bovine serum), the infected cells (RAW 264.7 cells) were preliminarily seeded in a 24-well plate for tissue culture at the cell number of 5 × 10⁵ cells per well, and cultured in a CO₂ incubator for 1 day under the culture conditions of 37°C and 5% CO₂. The cell growth culture medium was removed, 0.1 mL of the virus diluted solution was added to the wells to perform culture for 30 minutes to 1 hour, and thereafter the cells were overlaid with a culture medium for layering (DMEM culture medium to which 1.5% Sea Plaque Agarose (Lonza) was added).

After culturing in a CO₂ incubator for 2 days under the culture conditions of 37°C and 5% CO₂, 10% formalin was added to cause immobilization for 2 hours to during the night, and staining was performed with a gentian violet (crystal violet) solution containing 20% ethanol to measure the number of plaques. Two wells were tested under each condition to record the average number of plaques.

As a result, it was showed that the combination use of nattokinase and ε-polylysine with ethanol enhanced the anti- *Murine norovirus* activity (Table 18).

### [Table 18]

**Table 18**

| Test plot | Average number of plaque |
|---|---|
| Negative control | 65 |
| Nattokinase 0.1% + ε-polylysine 0.1% Ethanol 0% | 8.5 |
| Nattokinase 0.1% + ε-polylysine 0.1% Ethanol 50% | 0 |

### <Example 10> Anti-Feline calicivirus activity test at each pH (evaluation with log TCID₅₀ value)

The anti- *Feline calicivirus* activity at each pH was confirmed when subtilisin or a commercially available enzyme formulation containing a serine protease was used in combination with ε-polylysine. Enzyme formulations shown in Tables 19 to 22 were used and adjusted using PBS (-) (pH 6.0, pH 7.0 to 7.5, pH 9.0) in which the pH had been adjusted as shown in each table such that the concentration was a predetermined final concentration (volume percent concentration) of each test plot in each table to prepare serine protease solutions.

This test was conducted multiple times, and samples were respectively prepared at the concentrations shown in Tables 19 to 22.

### [Table 19]

**Table 19**

| Test plot | Serine protease | ε-Polylysine | Formulation pH |
|---|---|---|---|
| Negative control | 0 | - | - |
| Subtilisin | 20 ppm | 0.10% | 7.0 |
| Subtilisin | 20 ppm | 0.10% | 7.1 |
| Subtilisin | 20 ppm | 0.10% | 7.2 |
| Subtilisin | 20 ppm | 0.10% | 7.3 |
| Subtilisin | 20 ppm | 0.10% | 7.4 |
| Subtilisin | 20 ppm | 0.10% | 7.5 |
| Protin SD-AY10 | 250 ppm | 0.10% | 7.0 |
| Protin SD-AY10 | 250 ppm | 0.10% | 7.1 |
| Protin SD-AY10 | 250 ppm | 0.10% | 7.2 |
| Protin SD-AY10 | 250 ppm | 0.10% | 7.3 |
| Protin SD-AY10 | 250 ppm | 0.10% | 7.4 |
| Protin SD-AY10 | 250 ppm | 0.10% | 7.5 |
| Bioprase (registered trademark) OP | 250 ppm | 0.10% | 7.0 |
| Bioprase (registered trademark) OP | 250 ppm | 0.10% | 7.1 |
| Bioprase (registered trademark) OP | 250 ppm | 0.10% | 7.2 |
| Bioprase (registered trademark) OP | 250 ppm | 0.10% | 7.3 |
| Bioprase (registered trademark) OP | 250 ppm | 0.10% | 7.4 |
| Bioprase (registered trademark) OP | 250 ppm | 0.10% | 7.5 |

### [Table 20]

**Table 20**

| Test plot | Serine protease | ε-Polylysine | Formulation pH |
|---|---|---|---|
| Negative control | 0 | - | - |
| Bioprase (registered trademark) OP | 250 ppm | 0.10% | 6.0 |
| Bioprase (registered trademark) SP-20FG | 250 ppm | 0.10% | 6.0 |
| Protin SD-AY10 | 250 ppm | 0.10% | 6.0 |
| Alcalase (registered trademark) 2.4 L FG | 250 ppm | 0.10% | 6.0 |

### [Table 21]

**Table 21**

| Test plot | Serine protease | ε-Polylysine | Formulation pH |
|---|---|---|---|
| Negative control | 0 | - | - |
| Bioprase (registered trademark) OP | 250 ppm | 0.10% | 9.0 |
| Bioprase (registered trademark) SP-20FG | 250 ppm | 0.10% | 9.0 |
| Protin SD-AY10 | 250 ppm | 0.10% | 9.0 |
| Alcalase (registered trademark) 2.4 L FG | 250 ppm | 0.10% | 9.0 |

### [Table 22]

**Table 22**

| Test plot | Serine protease | ε-Polylysine | Formulation pH |
|---|---|---|---|
| Negative control | 0 | - | - |
| P "Amano" 3SD | 250 ppm | 0.10% | 7.0 |
| P "Amano" 3SD | 250 ppm | 0.10% | 7.1 |
| P "Amano" 3SD | 250 ppm | 0.10% | 7.2 |
| P "Amano" 3SD | 250 ppm | 0.10% | 7.3 |
| P "Amano" 3SD | 250 ppm | 0.10% | 7.4 |
| P "Amano" 3SD | 250 ppm | 0.10% | 7.5 |
| Bioprase (registered trademark) APL-30 | 250 ppm | 0.10% | 7.0 |
| Bioprase (registered trademark) APL-30 | 250 ppm | 0.10% | 7.1 |
| Bioprase (registered trademark) APL-30 | 250 ppm | 0.10% | 7.2 |
| Bioprase (registered trademark) APL-30 | 250 ppm | 0.10% | 7.3 |
| Bioprase (registered trademark) APL-30 | 250 ppm | 0.10% | 7.4 |
| Bioprase (registered trademark) APL-30 | 250 ppm | 0.10% | 7.5 |
| Bioprase (registered trademark) 30G | 250 ppm | 0.10% | 7.0 |
| Bioprase (registered trademark) 30G | 250 ppm | 0.10% | 7.1 |
| Bioprase (registered trademark) 30G | 250 ppm | 0.10% | 7.2 |
| Bioprase (registered trademark) 30G | 250 ppm | 0.10% | 7.3 |
| Bioprase (registered trademark) 30G | 250 ppm | 0.10% | 7.4 |
| Bioprase (registered trademark) 30G | 250 ppm | 0.10% | 7.5 |

The virus stock solution of the *Feline calicivirus* solution used in Example 1 was appropriately diluted with PBS (-) (pH 7.4) such that the dilution ratio was 10 times or less and the log TCID₅₀ value of a negative control was not less than 6, and the virus stock solution and a serine protease solution were mixed at the volume ratio of 1:9 and incubated for 10 minutes at room temperature. After incubation, the culture supernatant was diluted with PBS (-) (pH 7.4) to create 10⁰-fold to 10⁹-fold serially diluted solutions.

Using a cell growth culture medium (Eagle's Minimum Essential Medium supplemented with 5% fetal bovine serum, Sigma-Aldrich), the infected cells (CRFK cells ATCC CCL-94) were preliminarily seeded in a 96-well plate for tissue culture (IWAKI microplate for cell culture, made of polystyrene, flat bottom) at the cell number of 2 × 10⁴ cells per well (0.05 mL of cell growth culture medium), and cultured in a CO₂ incubator under the culture conditions of 37°C and 5% CO₂. After incubation, 0.05 mL of the serially diluted solution was added per well and mixed without removing the culture medium from each well to inoculate the cells with the virus solution. The cells were cultured in a CO₂ incubator for 2 or 3 days under the culture conditions of 37°C and 5% CO₂, and the cytopathicity was confirmed by with a microscope to measure the log TCID₅₀ value by a method known to those skilled in the art.

When subtilisin was used, the log TCID₅₀ value decreased by 1.1 or more at pH 7.0, and decreased by 3.1 or more at pH 7.2 or higher as compared to the negative control (Table 23). In addition, when Protin SD-AY10 was used, the log TCID₅₀ value decreased by 1.6 or more at pH 7.0, and decreased by 2.1 or more at pH 7.1 or higher as compared to the negative control (Table 23). Furthermore, when Bioprase (registered trademark) OP was used, the log TCID₅₀ value decreased by 1.1 or more at pH 7.2, and decreased by 2.6 or more at pH 7.3 or higher as compared to the negative control (Table 23). Furthermore, when Bioprase (registered trademark) OP, Bioprase (registered trademark) SP-20FG, Protin SD-AY10, or Alcalase (registered trademark) 2.4 L FG were used, the log TCID₅₀ value decreased by 0.2 to 0.7 at pH 6.0 as compared to the negative control, showing that the antiviral activity was low in all the cases (Table 24), whereas the log TCID₅₀ value decreased by 3.7 to 4.0 at pH 9.0 as compared to the negative control, showing that the antiviral activity was high in all the cases (Table 25). Furthermore, when P "Amano" 3SD was used, the log TCID₅₀ value decreased by 1.8 or more at pH 7.2, and decreased by 2.3 or more at pH 7.3 or higher as compared to the negative control (Table 26). Furthermore, when Bioprase (registered trademark) APL-30 was used, the log TCID₅₀ value decreased by 1.3 or more at pH 7.0, and decreased by 2.3 or more at pH 7.1 or higher as compared to the negative control (Table 26). Furthermore, when Bioprase (registered trademark) 30G was used, the log TCID₅₀ value decreased by 1.6 or more at pH 7.3, and decreased by 3.1 or more at pH 7.4 or higher as compared to the negative control (Table 26). In other words, it was shown that it is preferable to appropriately adjust pH and to select a serine protease formulation according to usage applications since the case of subtilisin can be effectively used preferably at pH 6.0 or higher, more preferably at pH 7.0 to 11.0, and further preferably at pH 7.3 to 10.3, and the optimal ranges of pH for the antiviral activity are different and the levels of the activity are also different between various serine proteases and commercially available enzyme formulations containing various serine proteases.

### [Table 23]

**Table 23**

| Test plot | Formulation pH | logTCID₅₀/mL | Antiviral activity |
|---|---|---|---|
| Negative control | - | 6.2 | - |
| Subtilisin | 7.0 | 5.1 | 1.1 |
| Subtilisin | 7.1 | 4.3 | 1.9 |
| Subtilisin | 7.2 | 3.1 | 3.1 |
| Subtilisin | 7.3 | 2.8 | 3.4 |
| Subtilisin | 7.4 | 2.8 | 3.4 |
| Subtilisin | 7.5 | 2.8 | 3.4 |
| Protin SD-AY10 | 7.0 | 4.6 | 1.6 |
| Protin SD-AY10 | 7.1 | 4.1 | 2.1 |
| Protin SD-AY10 | 7.2 | 3.8 | 2.4 |
| Protin SD-AY10 | 7.3 | 3.8 | 2.4 |
| Protin SD-AY10 | 7.4 | 3.8 | 2.4 |
| Protin SD-AY10 | 7.5 | 3.8 | 2.4 |
| Bioprase (registered trademark) OP | 7.0 | 5.3 | 0.9 |
| Bioprase (registered trademark) OP | 7.1 | 5.3 | 0.9 |
| Bioprase (registered trademark) OP | 7.2 | 5.1 | 1.1 |
| Bioprase (registered trademark) or | 7.3 | 3.6 | 2.6 |
| Bioprase (registered trademark) OP | 7.4 | 2.8 | 3.4 |
| Bioprase (registered trademark) OP | 7.5 | 2.8 | 3.4 |

### [Table 24]

**Table 24**

| Test plot | Formulation pH | logTCID₅₀/mL | Antiviral activity |
|---|---|---|---|
| Negative control | - | 6.3 | - |
| Bioprase (registered trademark) OP | 6.0 | 6.1 | 0.2 |
| Bioprase (registered trademark) SP-20FG | 6.0 | 5.6 | 0.7 |
| Protin SD-AY10 | 6.0 | 5.6 | 0.7 |
| Alcalase (registered trademark) 2.4 L FG | 6.0 | 5.6 | 0.7 |

### [Table 25]

**Table 25**

| Test plot | Formulation pH | logTCID₅₀/mL | Antiviral activity |
|---|---|---|---|
| Negative control | - | 6.8 | - |
| Bioprase (registered trademark) OP | 9.0 | 2.8 | 4.0 |
| Bioprase (registered trademark) SP-20FG | 9.0 | 2.8 | 4.0 |
| Protin SD-AY10 | 9.0 | 3.1 | 3.7 |
| Alcalase (registered trademark) 2.4 L FG | 9.0 | 3.1 | 3.7 |

### [Table 26]

**Table 26**

| Test plot | Formulation pH | logTCID₅₀/mL | Antiviral activity |
|---|---|---|---|
| Negative control | - | 6.1 | - |
| P "Amano" 3SD | 7.0 | 6.3 | -0.2 |
| P "Amano" 3SD | 7.1 | 5.8 | 0.3 |
| P "Amano" 3SD | 7.2 | 4.3 | 1.8 |
| P "Amano" 3SD | 7.3 | 3.8 | 2.3 |
| P "Amano" 3SD | 7.4 | 3.8 | 2.3 |
| P "Amano" 3SD | 7.5 | 3.8 | 2.3 |
| Bioprase (registered trademark) APL-30 | 7.0 | 4.8 | 1.3 |
| Bioprase (registered trademark) APL-30 | 7.1 | 3.8 | 2.3 |
| Bioprase (registered trademark) APL-30 | 7.2 | 3.8 | 2.3 |
| Bioprase (registered trademark) APL-30 | 7.3 | 3.8 | 2.3 |
| Bioprase (registered trademark) APL-30 | 7.4 | 3.8 | 2.3 |
| Bioprase (registered trademark) APL-30 | 7.5 | 3.8 | 2.3 |
| Bioprase (registered trademark) 30G | 7.0 | 6.3 | -0.2 |
| Bioprase (registered trademark) 30G | 7.1 | 5.3 | 0.8 |
| Bioprase (registered trademark) 30G | 7.2 | 5.3 | 0.8 |
| Bioprase (registered trademark) 30G | 7.3 | 4.6 | 1.6 |
| Bioprase (registered trademark) 30G | 7.4 | 2.8 | 3.3 |
| Bioprase (registered trademark) 30G | 7.5 | 3.1 | 3.1 |

### [Industrial Applicability]

According to the present invention, a novel antiviral agent which exhibits an inactivation effect against non-enveloped type viruses and has high safety that enables application to the fingers and oral ingestion is provided, making the present invention extremely useful.

## Claims

1. An antiviral agent comprising:
a serine protease; and
a cationic polymer,
wherein the antiviral agent is used against non-enveloped type viruses.

2. The antiviral agent according to claim 1, wherein the serine protease is produced from a bacterium belonging to the genus *Bacillus,* a bacterium belonging to the genus *Flavobacterium,* a bacterium belonging to the genus *Arthrobacter,* a bacterium belonging to the genus *Streptomyces,* a bacterium belonging to the genus *Geobacillus,* a bacterium belonging to the genus *Aspergillus,* or a bacterium belonging to the genus *Rhizopus.*

3. The antiviral agent according to claim 1 or 2, wherein the serine protease is a protein having an amino acid sequence set forth in any one of SEQ ID NOs: 1 to 7, or a protein having an amino acid sequence that is 90% or more identical to the amino acid sequence set forth in any one of SEQ ID NOs: 1 to 7 and having an antiviral activity.

4. The antiviral agent according to any one of claims 1 to 3, wherein the serine protease is subtilisin or nattokinase.

5. The antiviral agent according to claim 1, wherein the serine protease is trypsin.

6. The antiviral agent according to any one of claims 1 to 5, wherein the cationic polymer is polylysine or polyethylenimine.

7. The antiviral agent according to any one of claims 1 to 6, wherein the non-enveloped type virus is *Norovirus.*

8. The antiviral agent according to any one of claims 1 to 7, further comprising an alcohol.

9. The antiviral agent according to any one of claims 1 to 8, wherein the antiviral agent is in a form of an aerosolized agent.

10. A textile product which is obtained by applying the antiviral agent according to any one of claims 1 to 8.

11. A food and drink composition comprising the antiviral agent according to any one of claims 1 to 8.

12. A food cleaning treatment agent comprising the antiviral agent according to any one of claims 1 to 8.
